# EUROPEAN PATENT APPLICATION

(11) **EP 3 391 863 A1**
(43) Date of publication of application: **24.10.2018**
(21) Application number: 18168083.6
(22) Date of filing: 18.04.2018
(51) Int. Cl.: A61F 13/42, A61F 13/00, A61F 13/49

(54) **MONITORING AND EVALUATION OF DIAPERS**

(30) Priority: 20.04.2017 EP 17167373
(71) Applicant: Open Care Group B.V., 6372 HC Landgraaf (NL)
(72) Inventor: VAN EKRIS, Valentijn, 6372 HC Landgraaf (NL); DE HAAN, Joshua, 6191 AA Beek (NL)
(74) Representative: Pronovem

(57) **Abstract**

An assembly for measuring a saturation level of a diaper, in particular incontinence or adult diaper, comprising an apparatus for detecting moisture and an attachment means for attaching a sensing element of the apparatus in proximity of the waist portion of the diaper.

## Description

### FIELD OF THE INVENTION

The present invention generally relates to an assembly for measuring the saturation level of a diaper, in particular incontinence or adult diaper. The present invention generally relates to a system for monitoring and evaluating diapers, in particular incontinence or adult diapers. The present invention further relates to a use of an assembly for measuring the saturation level of a diaper, in particular incontinence or adult diaper. In addition, the present invention generally relates to a method for monitoring and evaluating diapers, in particular incontinence or adult diapers. The invention is not limited to any particular type of diapers.

### BACKGROUND OF THE INVENTION

Diapers, in particular incontinence or adult diapers, are commonly used by people requiring incontinence aids that are not able to change incontinence material themselves and alert the environment in the case of an incontinence incident. This results in exposure of this person to urine and fecal matter, which produces discomfort. In case of prolonged exposure it may even lead to morbidity (e.g. urinary tract infection), which in untreated cases may also lead to mortality.

As a consequence these people require to be monitored carefully by caregivers for instance relatives or nurses. This becomes cumbersome in situations where multiple of these people are housed together and are monitored by a limited group of caregivers, like patients in institutions such as hospitals, nursing homes and geriatric institutions. Therefore, these institutions usually operate on the basis of routines and schedules that allow for an efficient and regular changing event typically once every hours, to limit the maximal duration of exposure. Furthermore, it is common that patients in these homes are provided with absorbent pull up pants with an absorbent capacity that considerably (e.g. oftentimes by as much as tenfold) exceeds the required capacity for the applied schedule.

The main disadvantage of this approach is that it leads to considerably more nursing time, waste and costs than is required, because absorbent pull up pants that are unused and not used up to the advised saturation level (i.e between 50% and 75% according to standard ISO 11948-1) are being changed. Furthermore, this approach does not prevent exposure of the patient to moisture from urine and/or fecal matter, since the incontinence incident can occur instantly after a changing event and the amount of expelled urine and fecal matter may change quite rapidly in time due to several factors like illness, dietary changes and weather conditions.

Various incontinence detectors for absorbent articles are known (e.g. US5903222A, US8866624B2). These devices typically detect moisture by altering characteristics, like conductivity and capacitance, in an electronic circuit and alert the caregiver when any moisture is detected without indicating the amount. The advantage is that these devices can help prevent the exposure of the patient to moisture from urine and/or fecal matter. The disadvantage is that incontinence incidents with small amounts of moisture or other events like perspiration may lead to a false alert, which can result in more nursing time than necessary, waste and costs.

US9283123B2 describes a system for monitoring subjects suffering from incontinence, wherein the volume of each wetness event is estimated. These estimated volumes can be accumulated to estimate the total volume of all wetness events after a changing event. The estimated total volume in combination with the capacity of the absorbent article that is worn by the patient can be used to indicate when a changing event is recommended. Furthermore, the historical data of each patient collected by this system can be used to develop an incontinence care plan comprising selecting the appropriate capacity of the absorbent article and accordingly plan an appropriate changing moment. Furthermore, it can be used by institutions as input for supply management.

One disadvantage of a system according to US9283123B2 is that it requires various inputs to function, like the moment that a changing event occurs, the capacity of the worn incontinence material. Furthermore, there are no fall back safeguards such as in the event that the inputs are erroneous, the detector temporarily dysfunctions or disconnects, the estimation algorithm is false. A further disadvantage is that a system that calculates the total estimated volume of all wetness events by accumulating the estimated volume of each wetness event, is that the detector at least partly needs to be integrated near the site where moisture is secreted. This either means that: a) some of the electronics of the detector have to be integrated in the absorbent article, which leads to extra costs or b) the detector comprises a multicomponent assembly, which adds to costs and limits the robustness or c) the detector is a single component device that produces discomfort.

### OBJECT OF THE INVENTION

It is an object of the present invention to provide an apparatus, system and method for monitoring and evaluating diapers that is not subject to the described disadvantages, e.g. which is more robust.

### SUMMARY OF THE INVENTION

According to one aspect of the invention, there is therefore provided an assembly for measuring a saturation level of a diaper comprising an apparatus for detecting moisture and an attachment means for attaching a sensing element of the apparatus in proximity of the waist portion of the diaper. The apparatus for detecting moisture comprises a sensing element that is configured to detect a moisture level of an absorbing material of the diaper corresponding to a saturation level of the absorbing material of the diaper. The attachment means are advantageously arranged to position the sensing element at a predetermined position of the diaper. The predetermined position of the sensing element advantageously corresponds to a position of the diaper at which a predetermined saturation level of the absorbing material between 50% and 95%, more preferably between 65% and 85% and even more preferably between 70% and 80%, such as about 75% can be sensed. These saturation levels can advantageously be detected when the predetermined position of the sensing element is such that a moisture level of a portion of the absorbing material of the diaper arranged at preferably between 12 cm and 6 cm, more preferably between 11 cm and 7 cm and even more preferably at approximately 9 cm below the front waist portion of the diapers, e.g. below the waist band of the diaper, is sensed. To this end, the sensing element, e.g. a capacitor, is advantageously arranged at the above indicated distance below the waist and is advantageously arranged in close proximity to (an external surface or layer of) the diaper. An assembly according to aspects of the invention advantageously does not detect each incontinence incident or estimate the volume of the moisture for each incontinence incident, but rather detects the relevant saturation level parameter directly which therefore leads to a more robust system. The apparatus according to the invention detects reaching a predetermined saturation level between 50% and 95%, more preferably between 65% and 85% and even more preferably of approximately 75% at which changing diapers is advised by the industry. Using the de facto industry standard these saturation levels are typically reached when moisture reaches a predetermined distance between 12 cm and 6 cm, 11 cm and 7 cm, and 9 cm below the front waist portion of the diapers, respectively. An assembly according to the present invention therefore does not require various inputs to function, like the moment that a changing event occurs or the capacity of the worn incontinence material because it detects the relevant chosen saturation level of 75% for example as it occurs and can then provide a signal to the caregiver. Furthermore, there is no risk with respect to erroneous inputs, the temporary dysfunction or disconnection of the apparatus or a false estimation algorithm. A further advantage is that an assembly according to the invention can be fully integrated and be located at a comfortable location near the waist of the patient.

In a preferred embodiment, the attachment means comprise a grasping or a clipping mechanism arranged to be attached to the waist portion of the diaper, the waist band of other clothing articles or a belt. Advantageously, the apparatus for detecting moisture, and in particular the sensing element, is attached to the attachment means through an advantageously resilient member. The member extends from the attachment means to the sensing element such that the sensing element can be correctly positioned to enable measuring a desired saturation level as indicated above.

The sensing element of the apparatus for detecting moisture may be any sensing component capable of detecting a dielectric fluid like urine, these include a conductor, an electrical resistance and an inductive element. In a preferred embodiment, the sensing element comprises at least one capacitor arranged to change capacitance when the apparatus is near moisture. This moisture detection by means of capacitance does not require direct contact between the apparatus or sensing element of the apparatus and the moisture itself. Therefore, the apparatus stays clean during use. Furthermore, because no electric components are exposed to the external environment it is easier to clean when not in use, for instance during a changing event.

In a practical embodiment, the apparatus comprises an array of sensing elements, e.g. capacitors, that are arranged to detect different saturation levels. Such an array allows the selection of a saturation level at which the environment is notified and can be used to predict when a changing event would be required.

Preferably, the capacitor comprises a coplanar-plate capacitor. The electric field generated by these coplanar-plate capacitors stray into the environment much more than the electric fields that are generated by parallel-plate capacitors and are therefore influenced to a greater extent by a dielectric fluid (e.g. urine) near the capacitor.

In a practical embodiment, the apparatus comprises two surfaces substantially parallel to the coplanar-plate capacitor, wherein one of the two surfaces is arranged to face the diaper and wherein the other one of the two surfaces is arranged opposite the one surface and facing the external environment (i.e. facing away from the diaper). Preferably, the surface facing the diaper is arranged to enable sensing moisture and the surface facing the external environment is arranged to provide capacitive shielding. This shielding prevents that the environment can influence moisture detection.

The housing of the apparatus is preferably arranged to be waterproof and splashproof and even more preferably to be waterproof and watertight. The housing can be composed of any non-metallic and non-conductive material. Preferably, it substantially comprises a plastic, which beneficially can be disinfected.

The apparatus advantageously further comprises communication means configured for operative connection to the sensing element. The communication means may be operable to read out the sensing element. The communication means of the apparatus may comprise any means suitable for notifying the environment that the diapers have reached the predetermined saturation limit. These may comprise direct communication means like audible alarms, visible alarms and haptic feedback means. In a preferred embodiment of the invention, the apparatus comprises indirect communication means like WiFi, Bluetooth, 2G/3G/4G etc. that are suitable to wirelessly transmit a signal to a user-terminal, like a server, mobile device or any other receiver that the caregiver can interact with. Such a system for monitoring and evaluating diapers comprising at least one user-terminal equipped with indirect communication means and at least one apparatus equipped with indirect communication means allows the communication between the at least one user-terminal and the at least one apparatus. This user-terminal can be used to develop an incontinence care plan comprising selecting the appropriate capacity of the absorbent article and accordingly plan an appropriate changing moment. Furthermore, it can be used by institutions as input for supply management.

According to another aspect of the invention, there is provided a use of the above assembly for monitoring and evaluation of diapers, comprising applying the apparatus such that the moisture sensing elements are arranged to detect a saturation level of between 50% and 95%, more preferably between 65% and 85% and even more preferably of approximately 75%. This can be achieved when the moisture sensing elements are positioned between 12 cm and 6 cm, 11 cm and 7 cm, and 9 cm below the front waist portion of the diapers.

According to another aspect of the invention, there is provided a method for monitoring and evaluation of diapers, the method comprising:
- selecting a wearing time period for diapers, the diapers comprising absorbing materials having a first moisture absorbing capacity, the diapers being changed at changing events occurring upon expiry of the wearing time period,
- at each changing event, attaching a moisture sensing apparatus (e.g. the assembly as described above) to the diaper at a predetermined position corresponding to a saturation level of the absorbing material of the respective diaper,
- monitoring the moisture sensing apparatus and, upon the moisture sensing apparatus detecting a saturation level, recording a time elapsed to reach the saturation level and determining a second moisture absorbing capacity of the absorbing material of the diaper on the basis of the time elapsed to reach the saturation level.

The first capacity can have any desired capacity. Preferably, a calibration capacity is chosen which is relatively small for the wearing time period. This enables to quickly determine a second capacity that is more suitable for the chosen wearing time period. This second capacity can be chosen based on a function that uses historical data of the patient, the population, epidemiologic data, available diaper capacities and other types of data that may be relevant to choose a suitable capacity. For instance, the new capacity can be determined based on a function factoring in the wearing time period, the first capacity and the time it took to reach the saturation level of the first capacity.

One advantage of methods according to aspects of the invention is that the absolute moisture absorbing capacity of the absorbing material need not be known for the method to function properly. It may be sufficient for the method to function when it is known how the absorbing materials of different diapers are ranked in terms of moisture absorbing capacity. This will make it much easier for an operator to use the method.

Advantageously, the wearing time period is a fixed time period throughout the different changing events. Advantageously, the moisture sensing apparatus is reused at each changing event.

Preferably, the method also comprises determining a third capacity of the absorbing material of the diaper smaller than the first or second capacity determined at a preceding changing event, if, upon expiry of the wearing time period, no saturation level was detected by the moisture sensing apparatus. This also enable the lowering of the chosen capacity when the saturation is never reached before the end of the wearing time period. This can for instance occur after a period of illness, when urine secretion was higher than regular for this patient. In a practical embodiment, the functions that determine the suitable capacity are arranged to limit the continuous changing of capacity, which may occur when the saturation levels of two consecutive capacity levels are relatively far apart.

### BRIEF DESCRIPTION OF THE DRAWINGS

The invention will now be explained in greater detail with reference to the figures, in which equal or similar parts are indicated by the same reference signs, and in which:
Figures 1, 3 and 4 show perspective views of various preferred embodiments of an assembly according to the invention; and
Figures 2, 5 and 6 show various perspective views of the assembly of figs. 1, 3 and 4 respectively in use and attached to a diaper; and

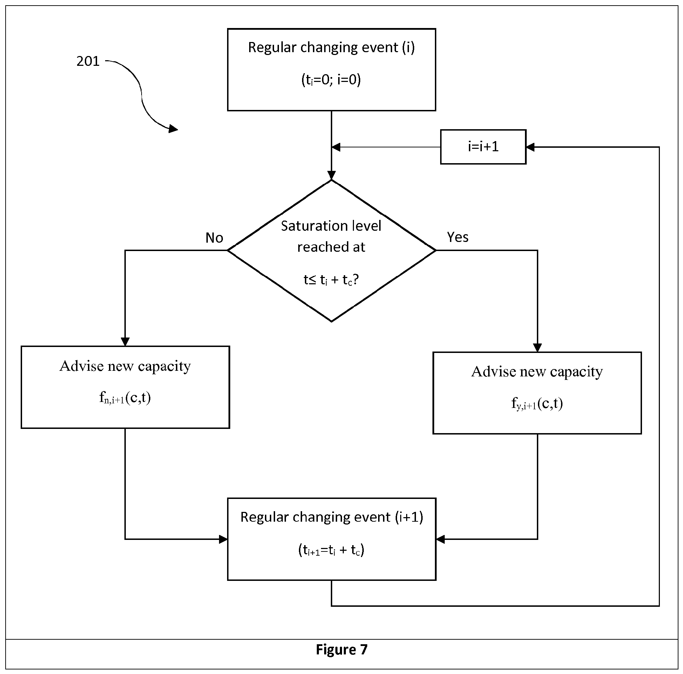

Figure 7 shows a schematic representation of a method according to the invention.

### DETAILED DESCRIPTION

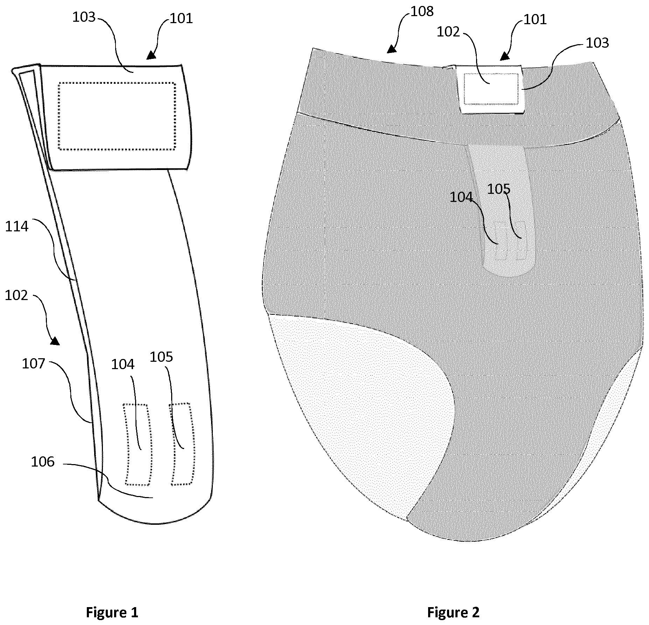

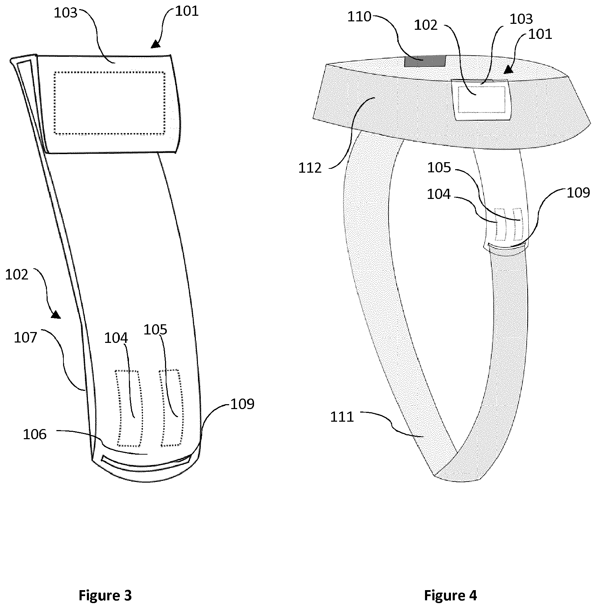

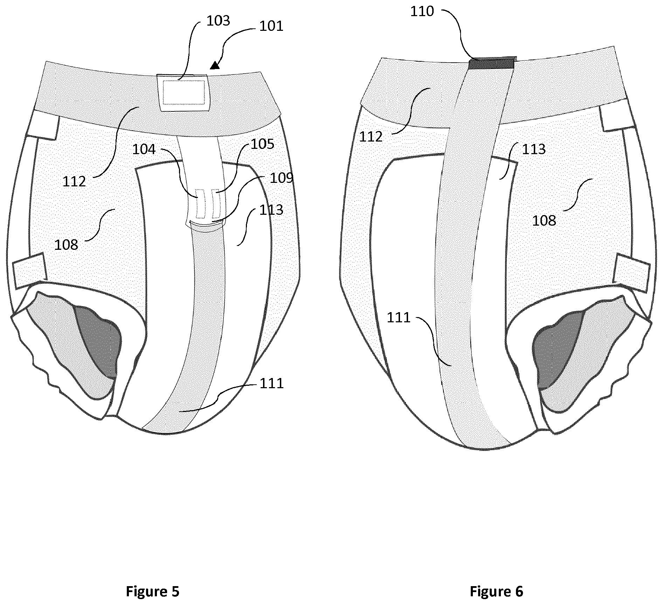

Figures 1, 3 and 4 relate to an assembly 101 according to preferred embodiments of the present invention. Figures 2, 5 and 6 relate to various views of the assembly 101 according to the invention in use. Figure 6 relates to a preferred method 201 according to the invention.

The assembly 101 for measuring a saturation level of a diaper 108 comprises an apparatus 102 for detecting moisture in the absorbing material 113 of diaper 108 near the sensing elements 104, 105. The assembly 101 further comprises attachment means 103 for attaching the apparatus 102 to the diaper 108, at a predetermined position thereof. Apparatus 102 may e.g. be attached at a distal end of a resilient member 114, which member is attached to the attachment means 103 at its proximal end. The length of member 114 may determine a desired position on the diaper 108. The sensing elements 104, 105 are advantageously externally attached to an external or cover layer of diaper 108 in order not to be in direct contact with the absorbing material 113. Advantageously, an impermeable cover layer is interposed between the absorbing material 113 and the sensing elements 104, 105. The attachment means 103 can for instance be attached to a piece of underwear 108 or a belt 112. The attachment means 103 are arranged at a distance from the sensing elements 104, 105 such that the sensing elements 104, 105 are located at a distance below the waist (e.g. 9 cm) that corresponds to a relevant saturation level (e.g. 75%). The sensing elements 104, 105 can be co-planar capacitive plates, which are unshielded at the surface 107 arranged to face the diaper, which thereby does not obstruct sensing the moisture. These plates may be shielded from the external environment on a surface 106 opposite surface 107 to limit the influence of external factors on moisture detection.

The assembly 101 may also be equipped with a fixation means 109 to fixate the end opposite to the attachment means. This fixation means 109 can for instance be provided by an opening for attaching a strap or an elastic band 111 that is equipped with a fastening means 110 for fastening the other end of the strap or elastic band 111 to the belt 112 or waist portion of the underwear 108.

## Claims

1. An assembly for measuring a saturation level of a diaper, in particular incontinence or adult diaper, comprising an apparatus for detecting moisture and an attachment means for attaching a sensing element of the apparatus in proximity of the waist portion of the diaper.

2. Assembly according to claim 1, wherein the sensing element is configured to detect a moisture level of an absorbing material of the diaper corresponding to a saturation level of the absorbing material of the diaper.

3. Assembly according to claim 1 or 2, wherein the attachment means are arranged to position the sensing element at a predetermined position of the diaper.

4. Assembly according to claim 3, wherein the predetermined position is between 12 cm and 6 cm, more preferably between 11 cm and 7 cm and even more preferably at approximately 9 cm below the front waist portion of the diapers.

5. Assembly according to anyone of claims 1-4, wherein the attachment means comprise a grasping or a clipping mechanism arranged to be attached to the waist portion of the diaper, the waist band of other clothing articles or a belt.

6. Assembly according to anyone of claims 1-5, wherein the sensing element comprises at least one capacitor arranged to change capacitance when the apparatus is near moisture.

7. Assembly according to claim 6, wherein the at least one capacitor is a coplanar-plate capacitor.

8. Assembly according to claims 6 or 7, wherein the apparatus comprises two surfaces substantially parallel to the coplanar-plate capacitor, wherein one surface is arranged to face the diaper and wherein another surface is arranged to face the external environment and provides capacitive shielding.

9. Assembly according to anyone of 1-8, wherein the assembly comprises communication means for operative connection to the sensing element and operable to acquire a readout of the sensing element.

10. A system for monitoring and evaluating diapers, in particular incontinence or adult diapers, comprising the assembly according to claim 9 and a user terminal.

11. A use of the assembly of any one of the claims 1 to 9, or the system of claim 10 for measuring the saturation level of a diaper, in particular incontinence or adult diaper.

12. Use according to claim 11, wherein the moisture sensing elements are positioned between 12 cm and 6 cm, preferably 11 cm and 7 cm, and more preferably 9 cm below the front waist portion of the diapers.

13. A method for monitoring and evaluating diapers, in particular incontinence or adult diapers, comprising:
selecting a wearing time period for diapers, the diapers comprising absorbing materials having a first capacity, the diapers being changed at changing events occurring upon expiry of the wearing time period,
at each changing event, attaching a moisture sensing apparatus to the diaper at a predetermined position corresponding to a saturation level of the absorbing material of the respective diaper,
monitoring the moisture sensing apparatus and, upon the moisture sensing apparatus detecting a saturation level, recording a time elapsed to reach the saturation level and determining a second capacity of the absorbing material of the diaper on the basis of the time elapsed to reach the saturation level.

14. Method according to claim 13 comprising determining a third capacity of the absorbing material of the diaper smaller than a second capacity determined at a preceding changing event, if, upon expiry of the wearing time period, no saturation level was detected by the moisture sensing apparatus.
